(19) Europäisches Patentamt
European Patent Office
Office européen des brevets

(11) EP 4 332 081 B1

(12) **EUROPEAN PATENT SPECIFICATION**

(45) Date of publication and mention of the grant of the patent:
**29.07.2026 Bulletin 2026/31**

(21) Application number: **22795828.7**

(22) Date of filing: **27.04.2022**

(51) International Patent Classification (IPC):
***C07C 67/62*** (2006.01) ***C07C 69/54*** (2006.01)
***C08F 2/44*** (2006.01) ***C08F 220/14*** (2006.01)

(52) Cooperative Patent Classification (CPC):
(C-Sets available)
**C07C 67/62; C08F 2/44; C08F 220/14** (Cont.)

(86) International application number:
**PCT/JP2022/019006**

(87) International publication number:
**WO 2022/230915 (03.11.2022 Gazette 2022/44)**

(54) **METHYL METHACRYLATE-CONTAINING COMPOSITION AND METHYL METHACRYLATE POLYMER PRODUCTION METHOD**

METHYLMETHACRYLATHALTIGE ZUSAMMENSETZUNG UND VERFAHREN ZUR HERSTELLUNG VON METHYLMETHACRYLATPOLYMER

COMPOSITION À TENEUR EN MÉTHACRYLATE DE MÉTHYLE, ET PROCÉDÉ DE FABRICATION DE POLYMÈRE DE MÉTHACRYLATE DE MÉTHYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2021 JP 2021075629**

(43) Date of publication of application:
**06.03.2024 Bulletin 2024/10**

(73) Proprietor: **Mitsubishi Chemical Corporation**
**Chiyoda-ku**
**Tokyo 100-8251 (JP)**

(72) Inventors:
- **KURIHARA, Yu**
**Tokyo 100-8251 (JP)**
- **KATO, Yuki**
**Tokyo 100-8251 (JP)**
- **SUZUKI, Tatsuya**
**Tokyo 100-8251 (JP)**
- **NINOMIYA, Wataru**
**Tokyo 100-8251 (JP)**

(74) Representative: **Hoffmann Eitle**
**Patent- und Rechtsanwälte PartmbB**
**Arabellastraße 30**
**81925 München (DE)**

(56) References cited:
**CN-A- 1 331 669**
**JP-A- 2001 517 680**
**JP-A- 2007 045 803**
**JP-A- 2019 127 436**
**JP-A- S5 230 887**
**US-A- 3 356 663**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 67/62, C07C 69/54**

## Description

### TECHNICAL FIELD

The present invention relates to a methyl methacrylate-containing composition and a method of producing methyl methacrylate polymer.

### BACKGROUND ART

Methyl methacrylate (hereinafter also referred to as "MMA") is known to be an extremely useful substance used as a raw material of various applications and types of polymers. For example, polymethyl methacrylate, which is a homopolymer of methyl methacrylate, is used in signboards, lighting equipment, automotive parts, construction-related materials, light guiding panels for flat displays, light diffusion plates, and the like, taking advantage of its excellent transparency, weather resistance, and other properties. In addition, copolymers of methyl methacrylate and other monomers are used in paints, adhesives, resin modifier, artificial marble, latices for paper, and the like. Various methods have been developed for industrially producing methyl methacrylate, and for example, the acetone cyanohydrin (ACH) method, the new acetone cyanohydrin (new ACH) method, the C4 direct oxidation method, the direct methyl esterification method, the ethylene method, and the new ethylene method are known (Non-Patent Document 1). In these production methods, methyl methacrylate of a quality suitable for the intended use is obtained by carrying out purification such as distillation to remove unreacted raw materials and by-products contained in the produced methyl methacrylate.

Since methyl methacrylate has a tendency to polymerize, it is known that the quality of methyl methacrylate is maintained by adding a polymerization inhibitor when producing methyl methacrylate or storing produced methyl methacrylate (Non-Patent Document 2). For example, Patent Document 1 describes that methyl ether of hydroquinone (MEHQ) is particularly preferable among various polymerization inhibitors. Patent Document 2 describes that N,N'-dialkyl-p-phenylenediamine and N-oxyl are preferable among various polymerization inhibitors. Patent Document 3 describes distillation of methyl methacrylate in the presence of a phenol polymerization inhibitor. Patent Document 4 describes use of a diphenylamine derivative as a polymerization inhibitor. Patent Document 5 describes use of a benzene triamine derivative as a polymerization inhibitor.

Patent Document 6 describes a composition for use as a radical polymerization inhibitor of an ethylenically unsaturated aliphatic monomer and a process for preventing radical polymerization of these unsaturated monomers during industrial preparation.

Patent Document 7 describes a method for inhibiting polymerization of a vinyl monomer, a composition for inhibiting polymerization, and a method for producing a vinyl monomer using the composition.

Patent Document 8 describes a method for purifying methyl methacrylate. More specifically, it aims at providing a methyl methacrylate without color, and a resin or a method for obtaining a resin composition.

### PRIOR ART DOCUMENTS

### PATENT DOCUMENTS

Patent Document 1: JP 2004-155757 A
Patent Document 2: JP 2005-502695 A
Patent Document 3: JP H10-504553 A
Patent Document 4: JP 2002-533309 A
Patent Document 5: JP 2002-513034 A
Patent Document 6: CN 1 331 669
Patent Document 7: JP 2019 127436
Patent Document 8: JP 2007 045803


### NON-PATENT DOCUMENTS


Non-Patent Document 1: Toru Kuroda, "Development of Catalyst for Producing Methyl Methacrylate", Catalysts & Catalysis, 45 (5), 366-371, 2003, Catalysis Society of Japan

Non-Patent Document 2: Takayuki Otsu, "On the Functions of Polymerization Inhibitors", Synthetic Organic Chemistry, 33 (8), 634-640, 1975, The Society of Synthetic Organic Chemistry, Japan

## SUMMARY OF THE INVENTION

### PROBLEMS TO BE SOLVED BY THE INVENTION

However, even in cases where polymerization inhibitors as described above are added, the quality of methyl methacrylate may deteriorate during storage. A polymerization inhibitor can inhibit the polymerization reaction of methyl methacrylate during storage, but deterioration of the quality of methyl methacrylate may also be caused by other reactions. In view of the above circumstances, the purpose of the present invention is to provide a methyl methacrylate-containing composition with high quality stability during storage.

### MEANS FOR SOLVING THE PROBLEMS

In order to achieve the above purposes, the present inventors have intensively studied. As a result, they found that, in methyl methacrylate with the quality deteriorated during storage, the concentration of methyl methacrylate is reduced, and methyl methacrylate dimers and methyl pyruvate are generated. The presence of methyl methacrylate dimers in methyl methacrylate may change the structure of methyl methacrylate polymer obtained by polymerization to adversely affect its physical properties. Furthermore, the presence of methyl pyruvate in methyl methacrylate causes coloration of methyl methacrylate polymer obtained by polymerization. The present inventors have found that inclusion of an alkyl-substituted aryl compound represented by a specific structural formula in a methyl methacrylate-containing composition results in improved quality stability during storage, and reduced generation of methyl methacrylate dimers and methyl pyruvate, thereby completing the present invention.
Accordingly, the present invention provides the compositions and methods as claimed.

### EFFECT OF THE INVENTION

According to the present invention, a methyl methacrylate-containing composition having high quality stability with reduced generation of methyl methacrylate dimers and methyl pyruvate during storage can be provided.

## DETAILED DESCRIPTION OF THE INVENTION

Embodiments according to the present invention is described below.
As used herein, any numerical value range indicated by the term "to" means any range including the numerical values described before and after the term "to" as the lower and upper limit values, respectively, and "A to B" means A or more and B or less.

### [Methyl Methacrylate-containing Composition]

A methyl methacrylate-containing composition according to the present invention comprises methyl methacrylate, and an alkyl-substituted aryl compound represented by the following Formula (1) (component A), wherein the concentration of methyl methacrylate is from 99 to 99.99% by mass.

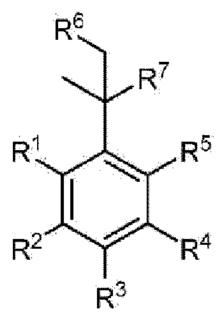

(1)

In Formula (1) above, $R^1$, $R^2$, $R^3$, $R^4$, $R^5$, and $R^6$ each independently represent a hydrogen atom, an alkyl group, an alkenyl group, an aryl group, a hydroxy group, an alkoxy group, an amino group, a monovalent group containing a carbonyl group, an alkylthio group, or an arylthio group. $R^7$ represents a hydrogen atom, an alkenyl group, an aryl group, a hydroxy group, an alkoxy group, an amino group, a monovalent group containing a carbonyl group, an alkylthio group, or an arylthio group.
The methyl methacrylate-containing composition may also comprise a polymerization inhibitor (component B),

other compound (component C), or water as long as the composition satisfies a concentration of methyl methacrylate of from 99 to 99.99% by mass. Each item is described below in detail.

(Component A)

The methyl methacrylate-containing composition according to the present invention comprises an alkyl-substituted aryl compound represented by the Formula (1) (component A). When the component A is included in the methyl methacrylate-containing composition, the generation of methyl methacrylate dimers and methyl pyruvate during storage of the methyl methacrylate-containing composition can be repressed. The reason for this is presumed as follows.

Methyl methacrylate dimers are generated due to radicals generated during storage of methyl methacrylate. Examples of the radicals include hydroxyl radicals generated when oxygen molecules absorb ultraviolet light from sunlight. Hydroxyl radicals also cause the generation of methyl pyruvate due to oxidation of methyl methacrylate. The aryl compound substituted with an alkyl group is a $\pi$-conjugated system compound having a benzene ring, and thus absorbs ultraviolet light. The absorption wavelength and absorption intensity vary depending on the type of the substituent. The aryl compound substituted with an alkyl group having a structure represented by the Formula (1) described above (component A) has an alkyl group having appropriate bulk and appropriate electron-donating properties bound to a benzene ring, and thus can absorb ultraviolet light across a broad wavelength. Therefore, when the methyl methacrylate-containing composition comprises a component A, a broad wavelength of ultraviolet light is absorbed, and the generation of hydroxyl radicals is reduced. It is estimated that this results in reduced generation of methyl methacrylate dimers and methyl pyruvate.

The molecular weight of the component A is preferably 2,000 or less. When the molecular weight is 2,000 or less, the number of benzene rings per unit mass in the component A can be increased, so that the effect of the present invention can be obtained with less mass. The molecular weight of the component A is more preferably 1,600 or less, still more preferably 1,200 or less, and particularly preferably 800 or less.

In the Formula (1), $R^1$, $R^2$, $R^3$, $R^4$, $R^5$, and $R^6$ each independently represent a hydrogen atom, an alkyl group, an alkenyl group, an aryl group, a hydroxy group, an alkoxy group, an amino group, a monovalent group containing a carbonyl group, an alkylthio group, or an arylthio group. $R^7$ in Formula (1) above represents a hydrogen atom, an alkenyl group, an aryl group, a hydroxy group, an alkoxy group, an amino group, a monovalent group containing a carbonyl group, an alkylthio group, or an arylthio group. $R^1$, $R^2$, $R^3$, $R^4$, $R^5$, $R^6$, and $R^7$ may be the same or different.

In the Formula (1), when $R^1$, $R^2$, $R^3$, $R^4$, $R^5$, $R^6$, and $R^7$ have large steric hindrance, the benzene ring is distorted, so that the n-conjugated system is not maintained. Conversely, when $R^1$, $R^2$, $R^3$, $R^4$, $R^5$, $R^6$, and $R^7$ satisfy the conditions described above, an appropriate size of bulk is achieved, and the n-conjugated system in the component A is maintained. Thus, the component A has a property of absorbing a broad wavelength of ultraviolet light, so that the effect of the present invention can be obtained. From the viewpoint of increasing the absorbance of ultraviolet light, and thereby reducing the generation of hydroxyl radicals, $R^1$, $R^2$, $R^3$, $R^4$, $R^5$, and $R^6$ are preferably a hydrogen atom, a $C_{1-5}$ alkyl group, a $C_{2-5}$ alkenyl group, a $C_{1-12}$ aryl group, a hydroxy group, a $C_{1-6}$ alkoxy group, an amino group, or a monovalent group containing a carbonyl group, more preferably a hydrogen atom, a $C_{1-5}$ alkyl group, a hydroxy group, or a $C_{1-6}$ alkoxy group, still more preferably a hydrogen atom, a methyl group, a t-butyl group, a hydroxy group, or a methoxy group, and particularly preferably a hydrogen atom, a methyl group, a hydroxy group, or a methoxy group. From the viewpoint of increasing the absorbance of ultraviolet light, and thereby reducing the generation of hydroxyl radicals, $R^7$ is preferably a hydrogen atom, a $C_{2-5}$ alkenyl group, a $C_{1-12}$ aryl group, a hydroxy group, a $C_{1-6}$ alkoxy group, an amino group, or a monovalent group containing a carbonyl group, more preferably a hydrogen atom, a carboxy group, or a $C_{2-6}$ alkoxy carbonyl group, and still more preferably a hydrogen atom, a carboxy group, or a methoxycarbonyl group.

The alkyl group is a chain (linear or branched) alkyl group or a cyclic alkyl group. The alkyl group is preferably a $C_{1-20}$ alkyl group, more preferably a $C_{1-10}$ alkyl group, and still more preferably a $C_{1-5}$ alkyl group. Examples of the chain alkyl group include a methyl group, an ethyl group, an n-propyl group, an isopropyl group, an n-butyl group, an isobutyl group, an s-butyl group, a t-butyl group, an n-pentyl group, an isopentyl group, a hexyl group, an octyl group, a decyl group, a hydroxymethyl group, a 1-hydroxyethyl group, and a 2-hydroxyethyl group; and a methyl group, an ethyl group, an n-propyl group, an isopropyl group, and t-butyl group are preferable. Examples of the cyclic alkyl group include a cyclopentyl group, a cyclohexyl group, and a cyclooctyl group.

The alkenyl group is a chain (linear or branched) alkenyl group or a cyclic alkenyl group. The alkenyl group is preferably a $C_{2-20}$ alkenyl group, more preferably a $C_{2-10}$ alkenyl group, and still more preferably a $C_{2-5}$ alkenyl group. Examples of the chain alkenyl group include a vinyl group, a 1-propenyl group, an isopropenyl group, a 2-butenyl group, a 1,3-butadienyl group, a 2-pentenyl group, and a 2-hexenyl group. Examples of the cyclic alkenyl group include a cyclopentenyl group and a cyclohexenyl group.

The aryl group is preferably a $C_{1-20}$ aryl group, and more preferably a $C_{1-12}$ aryl group. The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

The alkoxy group is preferably a $C_{1-20}$ alkoxy group, more preferably a $C_{1-10}$ alkoxy group, and still more preferably a $C_{1-6}$ alkoxy group. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group includes an amino group without a substituent on the nitrogen atom ($-NH_2$), and an amino group in which some or all of the hydrogen atoms bound to the nitrogen atom are substituted with carbon atoms. The number of carbon atoms in an amino group substituted with carbon atoms is preferably from 1 to 20, more preferably from 1 to 10, and still more preferably from 1 to 5. Examples of the amino group include an unsubstituted amino group, a methylamino group, an ethylamino group, a propylamino group, a butylamino group, a dimethylamino group, a diethylamino group, an anilino group, a toluidino group, an anisidino group, a diphenylamino group, and an N-methyl-N-phenylamino group.

Examples of the monovalent group containing a carbonyl group include a formyl group, an acyl group, a carboxy group, an amide group, an alkoxy carbonyl group, a thiocarboxy group, and a thioester group.

The acyl group is a substituent in which a carbonyl group is linked with an alkyl group, an alkenyl group, or an aryl group. The total number of carbon atoms derived from a carbonyl group (one) and derived from an alkyl group, alkenyl group, or an aryl group is preferably from 2 to 20, more preferably from 2 to 10, and still more preferably from 2 to 6. Examples of the acyl group include an acetyl group, a propionyl group, a butylcarbonyl group, a vinylcarbonyl group, and a benzoyl group.

The amide group includes an amide group without a substituent on the nitrogen atom ($-CONH_2$), and an amide group in which some or all of the hydrogen atoms bound to the nitrogen atom are substituted with carbon atoms. The number of carbon atoms in the amide group, which is the total number of carbon atoms derived from a carbonyl group (one) and the number of carbon atoms substituted on the nitrogen atom, is preferably from 1 to 20, more preferably from 1 to 10, and still more preferably from 1 to 5. Examples of the amide group include an unsubstituted amide group, an N-methylamide group, an N-ethylamide group, an N-phenylamide group, an N,N-dimethylamide group, and an N-methyl-N-phenylamide group.

The alkoxy carbonyl group is a substituent in which a carbonyl group and an alkoxy group are linked, and also referred to as an ester group. The total number of carbon atoms derived from a carbonyl group (one) and derived from an alkoxy group is preferably from 2 to 20, more preferably from 2 to 10, and still more preferably from 2 to 6. Examples of the alkoxy carbonyl group include a methoxycarbonyl group, an ethoxycarbonyl group, a butoxycarbonyl group, and a phenoxycarbonyl group.

The thioester group is a substituent in which a carbonyl group and an alkylthio group or an arylthio group are linked. The total number of carbon atoms derived from a carbonyl group (one) and derived from an alkylthio group or an arylthio group is preferably from 2 to 20, more preferably from 2 to 10, and still more preferably from 2 to 6. Examples of the thioester group include a methylthiocarbonyl group, an ethylthiocarbonyl group, a butylthiocarbonyl group, and a phenylthiocarbonyl group.

The monovalent group containing a carbonyl group may be a substituent in which one or more hydrogen(s) in an alkyl group is/are substituted with a carbonyl group(s). Examples of such a substituent include a 2-acetoxyethyl group, a 2-acetoethyl group, and a 2-(acetoacetoxy)ethyl group.

The alkylthio group is preferably a $C_{1-20}$ alkylthio group, more preferably a $C_{1-10}$ alkylthio group, and still more preferably a $C_{1-5}$ alkylthio group. Examples of the alkylthio group include a methylthio group, an ethylthio group, a propylthio group, and an isopropylthio group.

The arylthio group is preferably a $C_{1-20}$ arylthio group, more preferably a $C_{3-10}$ arylthio group, and still more preferably a $C_{6-10}$ arylthio group. Examples of the arylthio group include a phenylthio group, and a tolylthio group.

Among compounds that satisfy the conditions described above, the component A is preferably methyl 2-phenylisobutyrate, 2-phenylisobutyric acid, 2-isopropylhydroquinone, 2-isopropyl-4-methoxyphenol, 2-methyl-2-phenylpropionamide, methyl 2-(2-hydroxy-5-methoxyphenyl)-2-methylpropionate, or methyl 2-(2,5-dihydroxyphenyl)-2-methylpropionate, and more preferably methyl 2-phenylisobutyrate, 2-phenylisobutyric acid, 2-isopropylhydroquinone, or 2-isopropyl-4-methoxyphenol, from the viewpoint of quality stability of methyl methacrylate-containing composition during storage.

The component A may be one kind or two or more kinds.

(Component B)

The methyl methacrylate-containing composition according to the present invention preferably comprises a polymerization inhibitor (component B). As used herein, the term "polymerization inhibitor" means a compound having a function to inhibit the polymerization reaction of methyl methacrylate. Examples of the polymerization inhibitor include a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound. Inclusion of the component B is included, the progress of the polymerization reaction of methyl methacrylate due to a radical polymerization mechanism during storage of methyl methacrylate can be repressed. The component B can also trap hydroxyl radicals as described above generated during storage of methyl methacrylate. That is, when the methyl methacrylate-containing composition comprises the component A as well as a component B, the hydroxyl radical content can be reduced via two different mechanisms, one in which the component A can reduce generation of hydroxyl radicals, and the other in which the component B can remove generated hydroxyl radicals. Thus, it is considered that the generation of methyl methacrylate dimers and methyl pyruvate can be efficiently reduced.

Examples of the polymerization inhibitor that is a phenol compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylene-bis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino) phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and α-nitroso-β-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, dit-butyl-4-methoxyphenol, pyrogallol-1,2-dimethylether, and hydroquinone monobenzoate.

Examples of the tocopherol include α-tocopherol, and 2,3-dihydro-2,2-dimethyl-7-hydroxybenzofuran.

Examples of the polymerization inhibitor that is a quinone compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor that is a nitrobenzene compound include nitrobenzene, o-dinitrobenzene, m-dinitrobenzene, p-dinitrobenzene, 2,4-dinitrotoluene, dinitrodurene, and 2,2-diphenyl-1-picrylhydrazyl.

Examples of the polymerization inhibitor that is an N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethanoloxy-2,2,6,6-tetramethyl-piperidine-N-oxyl, 4-benzoyloxy-2,2,6,6-tetramethylpiperidine-N-oxyl, 2,2,5,5-tetramethyl-piperidine-N-oxyl, 3-amino-2,2,5,5-tetramethyl-piperidine-N-oxyl, 4,4',4''-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butylnitroxide, and di-tert-amylnitroxide.

Examples of the polymerization inhibitor that is an amine compound include N,N-diphenylamine, alkylated diphenylamine, 4,4'-dicumyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (the alkyl groups may be the same or different, and may each independently comprise 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-α-naphthylamine, N-phenyl-β-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor that is a phosphorus-containing compound include triphenylphosphine, triphenyl phosphite, triethyl phosphite, tris(isodecyl) phosphite, tris(tridecyl) phosphite, phenyl diisooctyl phosphite, phenyl diisodecyl phosphite, phenyl di(tridecyl) phosphite, diphenyl isooctyl phosphite, diphenyl isodecyl phosphite, diphenyl tridecyl phosphite, phosphonic acid [1,1-diphenyl-4,4'-diylbistetrakis-2,4-bis(1,1-dimethylethyl)phenyl] ester, triphenyl phosphite, tris(nonylphenyl) phosphite, 4,4'-isopropylidenediphenol alkyl phosphite, tris(2,4-di-tert-butylphenyl) phosphite, tris(biphenyl) phosphite, distearyl pentaerythritol diphosphite, di(2,4-di-tert-butylphenyl) pentaerythritol diphosphate, di(nonylphenyl) pentaerythritol diphosphite, phenyl bisphenol-A pentaerythritol diphosphite, tetra(tridecyl)-4,4'-butylidenebis(3-methyl-6-tertbutylphenol)diphosphite, hexa(tridecyl)-1,1,3-tris(2-methyl-4-hydroxy-5-tert-butylphenyl) butanetriphosphite, 3,5-di-tert-butyl-4-hydroxybenzyl phosphate diethyl ester, sodium-bis(4-tert-butylphenyl) phosphate, sodium-2,2'-methylene - bis(4,6-di-tert-butylphenyl)phosphate, and 1,3-bis(diphenoxyphosphonyloxy)benzene.

Examples of the polymerization inhibitor that is a sulfur-containing compound include diphenyl sulfide, phenothiazine, 3-oxophenothiazine, 5-oxophenothiazine, phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor that is an iron-containing compound include iron (III) chloride.

Examples of the polymerization inhibitor that is a copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor that is a manganese-containing compound include manganese dialkyl dithiocarbamate (the alkyl groups are each any of a methyl group, an ethyl group, a propyl group, and a butyl group, and may be the same or different), manganese diphenyl dithiocarbamate, manganese formate, manganese acetate, manganese octanoate, manganese naphthenate, manganese permanganate, and ethylenediaminetetraacetic acid manganese salt.

Among those described above, the component B is preferably at least one polymerization inhibitor(s) selected from the group consisting of a phenol compound, an N-oxyl compound, an amine compound, a phosphorus-containing compound, and a sulfur-containing compound, more preferably at least one polymerization inhibitor(s) selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine, and particularly preferably at least one polymerization inhibitor(s) selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, and phenothiazine, from the viewpoint of quality stability of the methyl methacrylate-containing composition during storage.

The component B may be one kind or two or more kinds.

In cases where the methyl methacrylate-containing composition comprises a compound that corresponds to both components A and B, the compound is considered as the component A. Thus, in the case where the methyl methacrylate-containing composition comprises the component A and the component B, it means that the composition comprises the component B other than the compound. In cases where two or more compounds that correspond to both components A and B are contained, the compound with the highest molar concentration in the methyl methacrylate composition is considered as the component A, and the other compound(s) is/are considered as the component(s) B.

(Concentrations of Component A and Component B)

When the concentration of the component A is MA (μmol/L) and the concentration of the component B is MB (μmol/L), MB/MA is preferably from 0.005 to 30, and more preferably 20 or less, from the viewpoint of efficiency in reducing the generation of methyl methacrylate dimers. The lower limit of MB/MA is more preferably 0.05 or more from the viewpoint of reducing the production of methyl pyruvate.

MA is preferably from 1 to 10,000 μmol/L. When MA is 1 μmol/L or more, the effect of reducing the generation of methyl methacrylate dimers and methyl pyruvate can be sufficiently obtained. When MA is 10,000 μmol/L or less, the impurity content after production of methyl methacrylate polymer by polymerization of the methyl methacrylate-containing composition according to the present invention is reduced, so that adverse effects on physical properties of the polymer can be prevented. The lower limit of MA is more preferably 10 μmol/L or more, and still more preferably 50 μmol/L or more. The upper limit of MA is more preferably 5,000 μmol/L or less, still more preferably 3,000 μmol/L or less, and particularly preferably 1,000 μmol/L or less.

MB is preferably from 1 to 7,000 μmol/L. When MB is 1 μmol/L or more, the effect of reducing the generation of methyl methacrylate dimers and methyl pyruvate can be sufficiently obtained. When MB is 7,000 μmol/L or less, the

impurity content after production of methyl methacrylate polymer by polymerization of the methyl methacrylate-containing composition according to the present invention is reduced, so that adverse effects on physical properties of the polymer can be prevented. The lower limit of MB is more preferably 10 μmol/L or more, and still more preferably 40 μmol/L or more. The upper limit of MB is more preferably 5,000 μmol/L or less, still more preferably 3,000 μmol/L or less, and particularly preferably 1,000 μmol/L or less.

(Concentration of Methyl Methacrylate)

The concentration of methyl methacrylate in the methyl methacrylate-containing composition according to the present invention is from 99 to 99.99% by mass. When the concentration of methyl methacrylate is 99% by mass or more, the impurity content after production of methyl methacrylate polymer by polymerization of the methyl methacrylate-containing composition according to the present invention is reduced, so that adverse effects on physical properties of the polymer can be prevented. When the concentration of methyl methacrylate is 99.99% by mass or less, the purification cost can be reduced. The lower limit of the concentration of methyl methacrylate is preferably 99.8% by mass or more.

(Component C)

The methyl methacrylate-containing composition according to the present invention may contain other compound (component C) as long as the concentration of methyl methacrylate of from 99 to 99.99% by mass is satisfied. The component C includes, for example, impurities generated during the production of methyl methacrylate. For example, methyl methacrylate may contain diacetyl as an impurity. From the viewpoint of reducing coloration of the methyl methacrylate-containing composition, the concentration of diacetyl is preferably 55 μmol/L or less, more preferably 20 μmol/L or less, still more preferably 10 μmol/L or less, and particularly preferably 1 μmol/L.

(Analysis of Methyl Methacrylate-containing Composition)

Inclusion of the component A, the component B, the component C, and water in the methyl methacrylate-containing composition can be determined, for example, by GC-MS measurement. When the GC-MS chart of the methyl methacrylate-containing composition has a peak at the same retention time as a reference material for the component A, and when the m/z value detected in the mass spectrum of the peak corresponds to the exact mass of the component A, it can be determined that the methyl methacrylate-containing composition comprises the component A. In cases where the reference material of the component A is not available, it can be determined that the peak is the component A peak when the mass spectrum peak pattern shown in the GC-MS chart of the methyl methacrylate-containing composition corresponds to the mass spectrum pattern of the component A recorded in the mass spectrum database. In other words, it can be determined that the methyl methacrylate-containing composition comprises the component A. Examples of the mass spectrum database include NIST20, NIST17, NIST14, and NIST14s. When detection by GC-MS measurement is impossible due to low volatility, LC-MS can be used for detection. Inclusion of a component B, a component C, and water can also be determined by the same method.

The concentration of methyl methacrylate can be determined, for example, by carrying out GC-FID measurement of the methyl methacrylate-containing composition, quantifying methyl methacrylate using the area normalization method, and correcting the resulting value using the water concentration determined with a Karl-Fisher moisture meter. The concentration of the component A can be determined, for example, by performing GC measurement of the methyl methacrylate-containing composition and using the internal standard method. When the reference material of the component A is not available, and thus cannot be quantified by the internal standard method, GC-FID measurement for any organic compound having known concentration is carried out under the same conditions as the methyl methacrylate-containing composition, and then the concentration of the component A can be calculated using the following equation:

$$\text{The concentration of the component A } (\mu\text{mol/L}) = \frac{N}{N_A} \times \frac{S_A}{S} \times \text{M}$$

In the equation, N is the number of carbon atoms that the organic compound having known concentration contains in one molecule; NA is the number of carbon atoms that the component A contains in one molecule; SA is the peak area of the component A, S is the peak area of the organic compound having known concentration, and M is the concentration (μmol/L) of the organic component having known concentration.

When quantification by GC measurement is impossible due to low volatility, a chromatography method such as LC can be used for quantification.

The concentrations of the component B and component C can be also determined by the same method as the component A described above.

Inclusion of water by the methyl methacrylate-containing composition and its concentration can be determined by the Karl-Fischer method.

[Method of Producing Methyl Methacrylate-containing Composition]

The method of producing the methyl methacrylate-containing composition according to the present invention may be a method in which a component A and a component B are added to methyl methacrylate. Methyl methacrylate to be used may be a commercially available product, or methyl methacrylate produced by known methods such as the acetone cyanohydrin (ACH) method, the new acetone cyanohydrin (new ACH) method, the C4 direct oxidation method, the direct methyl esterification method, the ethylene method, and the new ethylene method may be used. The component A and component B to be used may be commercially available products, or those synthesized by known methods may be used. When methyl methacrylate produced by a known method such as the acetone cyanohydrin (ACH) method, the new acetone cyanohydrin (new ACH) method, the C4 direct oxidation method, the direct methyl esterification method, the ethylene method, or the new ethylene method is used, the component A or the component B may be added to raw materials or in the course of the production process to produce the methyl methacrylate-containing composition. In cases where a component A or a component B is produced as a by-product during the methyl methacrylate production process, a part of the component A or the component B produced may be left for production of the methyl methacrylate-containing composition.

[Methods of Evaluating Storage Stability and Heat Stability]

The methyl methacrylate-containing composition according to the present invention has high quality stability during storage. The method of evaluating the quality stability of the methyl methacrylate-containing composition during storage may be, for example, a method in which the methyl methacrylate-containing composition is actually stored for a long period, and the amounts of methyl methacrylate dimers and methyl pyruvate generated are determined. From the viewpoint of work simplicity, a method may be used in which the methyl methacrylate-containing composition is heated for a short time, and the amounts of methyl methacrylate dimers and methyl pyruvate generated are determined. In cases of heating for short time, the heating temperature is preferably from 50 to 100°C, and the heating time period is preferably from 1 to 24 hours. In the present invention, the quality stability of the methyl methacrylate-containing composition during storage is evaluated based on the amounts of methyl methacrylate dimers and methyl pyruvate generated when the methyl methacrylate-containing composition is stored at 25°C for 14 days.

[Method of Producing Methyl Methacrylate Polymer]

The method of producing methyl methacrylate polymer according to the present invention comprises a step of polymerizing a polymeric composition comprising the methyl methacrylate-containing composition according to the present invention.

<Polymeric Composition>

The polymeric composition may comprise, as needed, a monomer that can be copolymerized with methyl methacrylate, and other additives.

(Monomer that can be copolymerized with Methyl Methacrylate)

Examples of the monomer that can be copolymerized with methyl methacrylate include the following:

a methacrylate ester, such as ethyl methacrylate, isopropyl methacrylate, n-butyl methacrylate, isobutyl methacrylate, tert-butyl methacrylate, 2-ethylhexyl methacrylate, phenyl methacrylate, or benzyl methacrylate;
an acrylate ester, such as methyl acrylate, ethyl acrylate, n-butyl acrylate, isobutyl acrylate, tert-butyl acrylate, or 2-ethylhexyl acrylate;
an unsaturated carboxylic acid, such as acrylic acid, methacrylic acid, maleic acid, or itaconic acid;
an unsaturated carboxylic anhydride, such as maleic anhydride, or itaconic anhydride;
maleimide, such as N-phenylmaleimide, or N-cyclohexylmaleimide;
a vinyl monomer containing a hydroxy group, such as 2-hydroxyethyl acrylate, 2-hydroxyethyl methacrylate, or 2-hydroxypropyl methacrylate;

a vinyl ester, such as vinyl acetate, or vinyl benzoate;
vinyl chloride, vinylidene chloride, and derivatives thereof;
a vinyl monomer containing nitrogen, such as methacrylamide, or acrylonitrile;
a monomer containing an epoxy group, such as glycidyl acrylate, or glycidyl methacrylate;
an aromatic vinyl monomer, such as styrene, or $\alpha$ - methylstyrene;
alkane diol di(meth)acrylate, such as ethylene glycol di(meth)acrylate, 1,2-propylene glycol di(meth)acrylate, 1,3-butyleneglycol di(meth)acrylate, or 1,6-hexanediol di(meth)acrylate;
polyoxyalkylene glycol di(meth)acrylate, such as diethylene glycol di(meth)acrylate, dipropylene glycol di(meth) acrylate, triethylene glycol (meth)acrylate, tetraethylene glycol di(meth)acrylate, polyethylene glycol di(meth)acrylate, or neopentyl glycol di(meth)acrylate;
a vinyl monomer having two or more ethylenic unsaturated bonds in its molecule, such as divinylbenzene;
an unsaturated polyester prepolymer obtained from at least one polyvalent carboxylic acid including ethylenic unsaturated polycarboxylic acid and at least one diol; and
a vinyl ester prepolymer obtained by acrylic modification of an end of an epoxy group.

Among those described above, the monomer that can be copolymerized with methyl methacrylate is preferably at least one selected from the group consisting of the methacrylate esters and the acrylate esters. This enables polymerization of the polymeric composition to obtain a methyl methacrylate polymer with excellent balance of transparency, heat resistance, and moldability. The monomer that can be copolymerized with methyl methacrylate is more preferably an acrylate ester, and particularly preferably at least one selected from the group consisting of methyl acrylate, ethyl acrylate, and n-butyl acrylate.
The monomer that can be copolymerized with methyl methacrylate may be one kind or two or more kind. When the component A is a monomer that can be copolymerized with methyl methacrylate, the component A may be used as the monomer that can be copolymerized with methyl methacrylate, or the monomer that can be copolymerized with methyl methacrylate apart from the component A may be used.
The content of the monomer that can be copolymerized with methyl methacrylate contained in the polymeric composition is preferably from 0 to 50 parts by mass with respect to 100 parts by mass of methyl methacrylate. This enables obtaining a methyl methacrylate polymer with high transparency. The upper limit of the content of the monomer that can be copolymerized with methyl methacrylate with respect to 100 parts by mass of methyl methacrylate is more preferably 40 parts by mass or less, and still more preferably 30 parts by mass or less. The lower limit of the content of the monomer that can be copolymerized with methyl methacrylate with respect to 100 parts by mass of methyl methacrylate is more preferably 0.01 parts by mass or more, still more preferably 0.1 parts by mass or more, and particularly preferably 1 part by mass or more.

(Other Additives)

Other additives preferably include a polymerization initiator. Other additives may also include, for example, a chain transfer agent, a mold release agent, a lubricant, a plasticizer, an antioxidant, an antistatic agent, a light stabilizer, an ultraviolet absorber, a flame retardant, a flame retardant promoter, a polymerization inhibitor, a filler, a pigment, a dye, a silane coupling agent, a leveling agent, an antifoam, and a fluorescent agent, as needed. The other additive may be one kind or two or more kinds.
Examples of the polymerization initiator include the following:

an azo compound, such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2-2'-azobis(2-methylpropane), 1,1-azobis(cyclohexanecarbonitrile), and dimethyl-2,2'-azobisisobutyrate;
an organic peroxide, such as benzoyl peroxide, 2,5-dimethyl-2,5-bis(t-butylperoxy)hexane, 1,1-bis(t-butylperoxy) cyclohexane, 1,1-bis(t-butylperoxy)-3,5,5-trimethylcyclohexane, t-butyl peroxy-2-ethylhexanoate, t-butyl peroxyisobutyrate, t-butyl peroxybenzoate, t-hexyl peroxybenzoate, t-butyl peroxyisopropyl monocarbonate, t-butyl peroxy-3,5,5-trimethyl hexanoate, t-butyl peroxylaurate, t-butyl peroxyacetate, t-hexyl peroxyisopropyl monocarbonate, t-hexyl peroxy-2-ethyl hexanoate, t-amyl peroxy-2-ethyl hexanoate, 1,1,3,3-tetramethyl butyl peroxyethyl hexanoate, 1,1,2-trimethyl propyl peroxy-2-ethyl hexanoate, 1,1,3,3-tetramethyl butyl peroxyisopropyl monocarbonate, 1,1,2-trimethyl propyl peroxyisopropyl monocarbonate, 1,1,3,3-tetramethyl butyl peroxyisononanoate, 1,1,2-trimethyl propyl peroxyisononanoate, di-t-butyl peroxide, di-t-hexyl peroxide, lauroyl peroxide, and dilauroyl peroxide;
a persulfate compound, such as potassium persulfate; and
a redox polymerization initiator.

Among those described above, the polymerization initiator is preferably at least one selected from the group

consisting of the azo compound and the organic peroxide from the viewpoint of storage stability and reactivity with methyl methacrylate.

The amount of the polymerization initiator used is preferably from 0.0001 to 1 part by mass with respect to 100 parts by mass of the total of methyl methacrylate and the monomer that can be copolymerized with methyl methacrylate.

<Method of Polymerizing Polymeric Composition>

Examples of the polymerization method include a bulk polymerization method, a solution polymerization method, an emulsion polymerization method, and a suspension polymerization method. From the viewpoint of environmental load due to the use of solvents and the like, and of transparency of the methyl methacrylate polymer to be obtained, a bulk polymerization method is preferable.

Specific means for the bulk polymerization method is not particularly limited, and a known casting polymerization method such as a cell casting method or a continuous casting method can be used for production.

The casting polymerization method is a method for obtaining methyl methacrylate polymer by injecting a polymeric composition into a mold composed of two inorganic glass plates or metal plates (for example, SUS plates) placed opposite each other at a predetermined distance with the periphery sealed with a gasket such as a soft resin tube, and allowing the composition to polymerize.

The mold for casting polymerization is not particularly limited, and known molds can be used. Examples of the mold for cell casting include those in which two plate-like bodies, such as inorganic glass plates, chromium-plated metal plates, and stainless steel plates, are placed opposite each other at a predetermined distance, and a gasket is placed around the periphery to allow the plate-like bodies and the gasket to form a sealed space. Examples of the mold for continuous casting include those in which a sealed space is formed by opposing faces of a pair of endless belts running in the same direction at the same speed, and gaskets running at the same speed as the endless belt on both sides of the endless belt.

The spacing of the void in a mold is adjusted as appropriate to obtain a resin sheet with a desired thickness, and is generally from 1 to 30 mm.

The polymerization temperature is preferably from 125 to 210°C. This enables achieving an appropriate polymerization rate. More preferably, the lower limit of the polymerization temperature is 130°C or more, and the upper limit is 180°C or less. The polymerization time is not particularly limited, and can be, for example, from 0.5 to 24 hours.

EXAMPLES

The present invention is described in detail below with reference to Examples and Comparative Examples, but is not limited to these Examples. Unless otherwise stated, the terms "%" and "ppm" in Examples and Comparative Examples mean "% by weight" and "ppm by weight," respectively.

The water concentration contained in a methyl methacrylate reagent was determined by the Karl-Fischer method. The composition of the components of the methyl methacrylate-containing composition before being stored was determined based on the amounts of the raw materials added. Methyl methacrylate dimers and methyl pyruvate in the methyl methacrylate-containing composition after being stored were determined by an absolute calibration curve method using GC-MS. The measurement conditions for GC-MS are shown below.

Instrument: GC-MS measuring system (product name: QP-2010SE, manufactured by Shimadzu Corporation)

[Conditions for GC]

Column (product name: DB-WAX, manufactured by Agilent Technologies, Inc.);
Length: 60 m, Inner diameter: 0.32 mm, Film thickness: 1.00 μm;
Injection volume: 1.0 μL;
Vaporizing chamber temperature: 210°C;
Column oven temperature: held at 35°C for 10 minutes, raised from 35°C to 150°C at 5 °C/min, held at 150°C for 17 minutes, raised from 150°C to 220°C at 5 °C/min, and held at 220°C for 6 minutes;
Carrier gas: helium;
Injection mode: split (split ratio: 50);
Control mode: constant linear velocity (25.0 cm/sec);
Pressure: 26.1 KPa;
Total flow: 52.5 mL/min;

Purge flow: 3.0 mL/min; and
Column flow: 0.97 mL/min.

[Conditions for MS]

Ionization method: EI (Electron Ionization);
Ion source temperature: 250°C;
m/z detection range: 10-300;
Detection time: 70 minutes; and
Interface temperature: 250°C.
Determination of the water concentration by the Karl-Fischer method was carried out using automated water measurement equipment (product name: AQV-2200, manufactured by Hiranuma Co., Ltd.).

(Example 1)

Using methyl 2-phenylisobutyrate as a component A, 0.0222 g of the component A was added to 10.0333 g of a methyl methacrylate reagent (water concentration: 240 ppm) to prepare a methyl methacrylate solution (A-1 solution). The concentration of the component A in the A-1 solution is shown in Table 1.

Using 2,4-dimethyl-6-t-butylphenol as a component B, 0.0829 g of the component B was added to 40.0221 g of a methyl methacrylate reagent (water concentration: 240 ppm) to prepare a methyl methacrylate solution (B-1 solution). The concentration of the component B in the B-1 solution is shown in Table 1.

Next, 0.1034 g of the A-1 solution and 0.1069 g of the B-1 solution were added to 20.0410 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored at 25°C for 14 days. The amounts of methyl methacrylate dimers and methyl pyruvate generated in the methyl methacrylate-containing composition after the storage are shown in Table 2.

(Examples 2 to 4)

A-1 solutions were prepared in the same manner as in Example 1 except that a compound shown in Table 1 was used as a component A, and the amounts of the methyl methacrylate reagent and the component A were changed as shown in Table 1.

B-1 solutions were prepared in the same manner as in Example 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate generated in the methyl methacrylate-containing compositions after the storage are shown in Table 2.

(Examples 5 to 11)

A-1 solutions were prepared in the same manner as in Example 1.

B-1 solutions were prepared in the same manner as in Example 1 except that a compound shown in Table 1 was used as a component B, and the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate generated in the methyl methacrylate-containing composition after the storage are shown in Table 2.

(Examples 12 and 13)

A-1 solutions and B-1 solutions were prepared in the same manner as in Example 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate produced in the methyl methacrylate-containing compositions after the storage are shown in Table 2.

(Example 14)

A B-1 solution was prepared in the same manner as in Example 1.

Next, using methyl 2-phenylisobutyrate as a component A, 0.0218 g of the component A and 0.1012 g of the B-1 solution were added to 20.0179 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Examples 15 and 16)

A-1 solutions and B-1 solutions were prepared in the same manner as in Example 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate generated in the methyl methacrylate-containing compositions after the storage are shown in Table 2.

(Example 17)

An A-1 solution was prepared in the same manner as in Example 1.

A B-1 solution was prepared in the same manner as in Example 1 except that the amounts of the methyl methacrylate reagent and the component B were changed as shown in Table 1.

Next, a methyl methacrylate-containing composition was prepared in the same manner as in Example 1 except that the amounts of the methyl methacrylate reagent, the A-1 solution, and the B-1 solution were changed as shown in Table 2. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Example 18)

An A-1 solution was prepared in the same manner as in Example 1.

Next, 0.1053 g of the A-1 solution was added to 20.0142 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Comparative Example 1)

A B-1 solution was prepared in the same manner as in Example 1.

Next, 0.2213 g of the B-1 solution was added to 40.0273 g of a methyl methacrylate reagent (water concentration: 240 ppm) to prepare a methyl methacrylate-containing composition. The concentrations of the components in the methyl

methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Comparative Example 2)

40.0000 g of a methyl methacrylate reagent (water concentration: 240 ppm) was used as a methyl methacrylate-containing composition and stored as in Example 1. The amounts of methyl methacrylate dimers and methyl pyruvate generated in the methyl methacrylate-containing composition after the storage are shown in Table 2.

(Comparative Example 3)

A B-1 solution was prepared in the same manner as in Example 1 except that the amounts of the methyl methacrylate reagent and the component B were changed as shown in Table 1.

Using diacetyl as a component C, 0.0205 g of the component C was added to 9.9913 g of a methyl methacrylate reagent (water concentration: 240 ppm) to prepare a methyl methacrylate solution (C-1 solution). The concentration of the component C in the C-1 solution is shown in Table 1.

Next, 0.2151 g of the B-1 solution and 0.2069 g of the C-1 solution were added to 40.0218 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Comparative Examples 4 and 5)

B-1 solutions were prepared in the same manner as in Example 1.

C-1 solutions were prepared in the same manner as in Comparative Example 3 except that a compound shown in Table 1 was used as a component C, and the amounts of the methyl methacrylate reagent and the component C were changed as shown in Table 1.

Next, a methyl methacrylate-containing composition was prepared in the same manner as in Comparative Example 3 except that the amounts of the methyl methacrylate reagent, the B-1 solution, and the C-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate generated in the methyl methacrylate-containing compositions after the storage are shown in Table 2.

(Comparative Example 6)

A B-1 solution was prepared in the same manner as in Example 1 except that the amounts of the methyl methacrylate reagent and the component B were changed as shown in Table 1.

Next, a methyl methacrylate-containing composition was prepared in the same manner as in Example 14 except that the amounts of the methyl methacrylate reagent, the component A, and the B-1 solution were changed as shown in Table 2. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Comparative Example 7)

An A-1 solution and a B-1 solution were prepared in the same manner as in Example 1.

Next, 0.1033 g of the A-1 solution, 0.1005 g of the B-1 solution, and 0.3218 g of pure water were added to 20.0179 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl

methacrylate dimers and methyl pyruvate generated in the methyl methacrylate-containing composition after the storage are shown in Table 2.

Table 1

| | A-1 solution | | | | B-1 solution | | | | C-1 solution | | | |
|---|---|---|---|---|---|---|---|---|---|---|---|---|
| | Amount of MMA regent added [g] | Component A | | | Amount of MMA regent added [g] | Component B | | | Amount of MMA regent added [g] | Component C | | |
| | | Compound name | Added amount [g] | Concent ration [ppm] | | Compound name | Added amount [g] | Concent ration [ppm] | | Compound name | Added amount [g] | Concent ration [ppm] |
| Example 1 | 10.0333 | Methyl 2-phenylisobutyrate | 0.0222 | 2208 | 40.0221 | 2,4-Dimethyl-6-t-butylphenol | 0.0829 | 2067 | - | - | - | - |
| Example 2 | 10.0254 | 2-Phenylisobutyric acid | 0.0212 | 2110 | 40.0221 | 2,4-Dimethyl-6-t-butylphenol | 0.0829 | 2067 | - | - | - | - |
| Example 3 | 10.0138 | 2-Isopropylhydroquinone | 0.0212 | 2113 | 40.0221 | 2,4-Dimethyl-6-t-butylphenol | 0.0829 | 2067 | - | - | - | - |
| Example 4 | 10.0333 | 2-Isopropyl-4-methoxyphenol | 0.0216 | 2148 | 40.0221 | 2,4-Dimethyl-6-t-butylphenol | 0.0829 | 2067 | - | - | - | - |
| Example 5 | 10.0333 | Methyl 2-phenylisobutyrate | 0.0222 | 2208 | 10.0051 | 4-Methoxyphenol | 0.0191 | 1905 | - | - | - | - |
| Example 6 | 10.0333 | Methyl 2-phenylisobutyrate | 0.0222 | 2208 | 10.0238 | Hydroquinone | 0.0233 | 2319 | - | - | - | - |
| Example 7 | 10.0333 | Methyl 2-phenylisobutyrate | 0.0222 | 2208 | 10.0204 | Phenothiazine | 0.0238 | 2370 | - | - | - | - |
| Example 8 | 10.0333 | Methyl 2-phenylisobutyrate | 0.0222 | 2208 | 10.0021 | 4-Hydroxy-2,2,6,6-tetramethylpiperidine-N-oxyl | 0.0202 | 2016 | - | - | - | - |
| Example 9 | 10.0333 | Methyl 2-phenylisobutyrate | 0.0222 | 2208 | 10.1244 | N,N-Diphenylamine | 0.0222 | 2188 | - | - | - | - |
| Example 10 | 10.0333 | Methyl 2-phenylisobutyrate | 0.0222 | 2208 | 10.0077 | N-Nitrosodiphenylamine | 0.0208 | 2074 | - | - | - | - |
| Example 11 | 10.0333 | Methyl 2-phenylisobutyrate | 0.0222 | 2208 | 10.0061 | Triphenyl phosphite | 0.0223 | 2224 | - | - | - | - |
| Example 12 | 10.0333 | Methyl 2-phenylisobutyrate | 0.0222 | 2208 | 40.0221 | 2,4-Dimethyl-6-t-butylphe-nol | 0.0829 | 2067 | - | - | - | - |

(continued)

| | A-1 solution | | | | B-1 solution | | | | C-1 solution | | | |
|---|---|---|---|---|---|---|---|---|---|---|---|---|
| | Amount of MMA regent added [g] | Component A | | | Amount of MMA regent added [g] | Component B | | | Amount of MMA regent added [g] | Component C | | |
| | | Compound name | Added amount [g] | Concent ration [ppm] | | Compound name | Added amount [g] | Concent ration [ppm] | | Compound name | Added amount [g] | Concent ration [ppm] |
| Example 13 | 10.0333 | Methyl 2-phenylisobutyrate | 0.0222 | 2208 | 40.0221 | 2,4-Dimethyl-6-t-butylphenol | 0.0829 | 2067 | - | - | - | - |
| Example 14 | - | - | - | - | 40.0221 | 2,4-Dimethyl-6-t-butylphenol | 0.0829 | 2067 | - | - | - | - |
| Example 15 | 10.0333 | Methyl 2-phenylisobutyrate | 0.0222 | 2208 | 40.0221 | 2,4-Dimethyl-6-t-butylphenol | 0.0829 | 2067 | - | - | - | - |
| Example 16 | 10.0333 | Methyl 2-phenylisobutyrate | 0.0222 | 2208 | 40.0221 | 2,4-Dimethyl-6-t-butylphenal | 0.0829 | 2067 | - | - | - | - |
| Example 17 | 10.0333 | Methyl 2-phenylisobutyrate | 0.0222 | 2208 | 40.0080 | 2,4-Dimethyl-6-t-butylphenol | 0.0828 | 2065 | - | - | - | - |
| Example 18 | 10.0333 | Methyl 2-phenylisobutyrate | 0.0222 | 2208 | - | - | - | - | - | - | - | - |
| Comparative example 1 | - | - | - | - | 10.0026 | 2,4-Dimethyl-6-t-butylohenol | 0.0228 | 2274 | - | - | - | - |
| Comparative example 2 | - | - | - | - | - | - | - | - | - | - | - | - |
| Comparative example 3 | - | - | | - | 10.0026 | 2,4-Dimethyl-6-t-butylphenol | 0.0228 | 2274 | 9.9913 | Diacetyl | 0.0205 | 2048 |
| Comparative example 4 | - | - | - | - | 40.0221 | 2,4-Dimethyl-6-t-butylphenol | 0.0829 | 2067 | 10.0170 | 2-t-Butyl hydroquinone | 0.0223 | 2221 |
| Comparative example 5 | - | - | - | - | 40.0221 | 2,4-Dimethyl-6-t-butylphenol | 0.0829 | 2067 | 10.0088 | 3-t-Butyl-4-hydroxyanisole | 0.0236 | 2352 |
| Comparative example 6 | - | - | - | - | 40.0080 | 2,4-Dimethyl-6-t-butylphenol | 0.0828 | 2065 | - | - | - | - |
| Comparative example 7 | 10.0333 | Methyl 2-phenylisobutyrate | 0.0222 | 2208 | 40.0221 | 2,4-Dimethyl-6-t-butylphenol | 0.0829 | 2067 | - | - | - | - |

Table 2

| | Added amount [g] | | | | | | MMA-containina composition | | | | | | | | Amount of MMA dimer generated [ppm] | Amount of methyl pyruvate generated [ppm] |
|---|---|---|---|---|---|---|---|---|---|---|---|---|---|---|---|---|
| | | | | | | | MMA | Component A | | Component B | | Component C | | MB/MA | | |
| | MMA reagent | A-1 solu-tion | B-1 solu-tion | C-1 solu-tion | Com-po nent A | Pure water | Con-cent ration [%] | Compound name | Concentra-tion MA [μmol/L] | Compound name | Concentra-tion MB [μmol/L] | Compound name | Con-cent ration [μmol/-L] | | | |
| Example 1 | 20.041-0 | 0.1034 | 0.1069 | - | - | - | 99.97 | Methyl 2-phenylisobutyrate | 60 | 2,4-Dimethyl-6-t-butylphenol | 58 | - | - | 0.968 | 16 | 2 |
| Example 2 | 20.016-5 | 0.1057 | 0.1050 | - | - | - | 99.97 | 2-Phenylisobutyric acid | 63 | 2,4-Dimethyl-6-t-butylphenol | 57 | - | - | 0.896 | 14 | 3 |
| Example 3 | 20.010-4 | 0.1033 | 0.1014 | - | - | - | 99.97 | Isopropylhydroquinone 2- | 67 | 2,4-Dimethyl-6-t-butylphenol | 55 | - | - | 0.820 | 21 | 0 |
| Example 4 | 20.027-4 | 0.1055 | 0.1030 | - | - | - | 99.97 | 2-Isopropyl-4-methoxyphenol | 64 | 2,4-Dimethyl-6-t-butylphenol | 56 | - | - | 0.876 | 13 | 0 |
| Example 5 | 20.006-9 | 0.1071 | 0.1057 | - | - | - | 99.97 | Methyl 2-phenylisobutyrate | 62 | 4-Methoxyphenol | 76 | - | - | 1.223 | 23 | 3 |
| Example 6 | 20.095-2 | 0.1091 | 0.1076 | - | - | - | 99.97 | Methyl 2 phenylisobutyrate | 63 | Hydroquinone | 105 | - | - | 1.677 | 19 | 0 |
| Example 7 | 20.037-1 | 0.1073 | 0.1062 | - | - | - | 99.97 | Methyl 2-phenylisobutyrate | 62 | Phenothiazine | 59 | - | - | 0.950 | 17 | 3 |
| Example 8 | 10.024-2 | 0.0525 | 0.0576 | - | - | - | 99.97 | Methyl 2-phenylisobutyrate | 61 | 4-Hydroxy-2,2,6,6-tetrameth ylpiperidine-N-oxyl | 63 | - | - | 1.036 | 7 | 9 |
| Example 9 | 10.027-7 | 0.0551 | 0.0524 | - | - | - | 99.97 | Methyl 2-phenylisobutyrate | 64 | N,N-Diphenylamine | 63 | - | - | 0.993 | 2 | 0 |
| Example 10 | 10.012-8 | 0.0526 | 0.0525 | - | - | - | 99.97 | Methyl 2 phenyliso-butyrate | 61 | N-Nitrosodiphenyla-mine | 51 | - | - | 0.843 | 3 | 0 |
| Example 11 | 10.026-8 | 0.0546 | 0.0544 | - | - | - | 99.97 | Methyl 2-phenylisobutyrate | 63 | Triphenyl phosphite | 36 | - | - | 0.576 | 6 | 19 |
| Example 12 | 19.012-7 | 1.0048 | 0.1031 | - | - | - | 99.96 | Methyl 2-phenylisobutyrate | 584 | 2,4-Dimethyl-6-t-butylphenol | 56 | - | - | 0.096 | 19 | 0 |

(continued)

| | Added amount [g] | | | | | | MMA-containina composition | | | | | | | | Amount of MMA dimer generated [ppm] | Amount of methyl pyruvate generated [ppm] |
|---|---|---|---|---|---|---|---|---|---|---|---|---|---|---|---|---|
| | | | | | | | MMA | Component A | | Component B | | Component C | | MB/MA | | |
| | MMA reagent | A-1 solution | B-1 solution | C-1 solution | Compo nent A | Pure water | Concentration [%] | Compound name | Concentration MA [µmol/L] | Compound name | Concentration MB [µmol/L] | Compound name | Concentration [µmol/L] | | | |
| Example 13 | 8.0070 | 2.0097 | 0.0523 | - | - | - | 99.93 | Methyl 2-phenylisobutyrate | 2334 | 2,4-Dimethyl-6-t-butylphenol | 57 | - | - | 0.024 | 18 | 2 |
| Example 14 | 20.0179 | - | 0.1012 | - | 0.0218 | - | 99.87 | Methyl 2-phenylisobutyrate | 5733 | 2,4-Dimethyl-6-t-butylphenol | 55 | - | - | 0.010 | 19 | 2 |
| Example 15 | 19.0303 | 0.1018 | 1.0013 | - | - | - | 99.96 | Methyl 2-phenylisobutyrate | 59 | 2,4-Dimethyl-6-t-butylphenol | 544 | - | - | 9.207 | 18 | 0 |
| Example 16 | 7.5119 | 0.0548 | 2.5081 | - | - | - | 99.92 | Methyl 2-phenylisobutyrate | 64 | 2,4-Dimethyl-6-t-butylphenol | 2725 | - | - | 42.840 | 23 | 1 |
| Example 17 | 4.0126 | 0.0504 | 6.0136 | - | - | - | 99.85 | Methyl 2-phenylisobutyrate | 58 | 2,4-Dimethyl-6-t-butylphenol | 6526 | - | - | 111.588 | 24 | 3 |
| Example 18 | 20.0142 | 0.1053 | - | - | - | - | 99.97 | Methyl 2-phenylisobutyrate | 61 | - | - | - | - | - | 17 | 3 |
| Comparative example 1 | 40.0273 | - | 0.2213 | - | - | - | 99.97 | - | - | 2,4-Dimethyl-6-t-butylphenol | 66 | - | - | - | 29 | 2 |
| Comparative example 2 | 40.0000 | - | - | - | - | - | 99.98 | - | - | - | - | - | - | - | 0 | 25 |
| Comparative example 3 | 40.0216 | - | 0.2151 | 0.2069 | - | - | 99.97 | - | - | 2,4-Dimethyl-6-t-butylphenol | 64 | Diacetyl | 115 | - | 0 | 1056 |
| Comparative example 4 | 40.0059 | - | 0.2063 | 0.2032 | - | - | 99.97 | - | - | 2,4-Dimethyl-6-t-butylphenol | 56 | 2-t-Butyl hydroquinone | 63 | - | 28 | 3 |
| Comparative example 5 | 40.0124 | - | 0.2041 | 0.2043 | - | - | 99.97 | - | - | 2,4-Dimethyl-6-t-butylphenol | 55 | 3-t-Butyl-4-hydroxyanisole | 62 | - | 29 | 0 |

(continued)

| | Added amount [g] | | | | | | MMA-containina composition | | | | | | | | Amount of MMA dimer generated [ppm] | Amount of methyl pyruvate generated [ppm] |
|---|---|---|---|---|---|---|---|---|---|---|---|---|---|---|---|---|
| | | | | | | | MMA | Component A | | Component B | | Component C | | MB/MA | | |
| | MMA reagent | A-1 solution | B-1 solution | C-1 solution | Compo nent A | Pure water | Concentration [%] | Compound name | Concentration MA [µmol/L] | Compound name | Concentration MB [µmol/L] | Compound name | Concentration [µmol/L] | | | |
| Comparative example 6 | 20.0120 | - | 0.1116 | - | 0.3080 | - | 98.47 | Methyl 2-phenylisobutyrate | 79843 | 2,4-Dimethyl-6-t-butylphenol | 60 | - | - | 0.001 | 34 | 9 |
| Comparative example 7 | 20.0179 | 0.1033 | 0.1005 | - | - | 0.3218 | 98.41 | Methyl 2-phenylisobutyrate | 59 | 2,4-Dimethyl-6-t-butylphenol | 54 | - | - | 0.911 | 31 | 4 |

As shown in Tables 1 and 2, Examples 1 to 18 in which the methyl methacrylate-containing composition contains a defined component A show reduction of both production of methyl methacrylate dimers and methyl pyruvate in the methyl methacrylate compositions after storage, and can be said to have high quality stability during storage. As compared to Example 18 only containing a component A, Example 1 further containing a component B in addition to a component A that was equivalent to that of Example 18 showed lower amount of methyl pyruvate produced. Thus, it can be said that including of both components A and B enables efficiently reducing the production of methyl methacrylate dimers and methyl pyruvate.

The polymeric composition comprising each methyl methacrylate-containing composition obtained in each Example can be polymerized to obtain a methyl methacrylate polymer.

INDUSTRIAL APPLICABILITY

According to the present invention, methyl methacrylate-containing compositions that can be used, for example, as raw materials for acrylic resins can be stored stably for a long period of time, which is industrially useful.

## Claims

1. A methyl methacrylate-containing composition, comprising:

   methyl methacrylate; and
   an alkyl-substituted aryl compound represented by the following Formula (1) (component A),
   wherein the concentration of methyl methacrylate is from 99 to 99.99% by mass,

   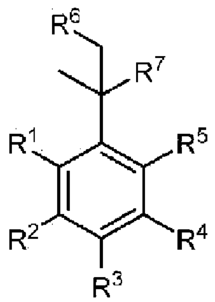

   (1)

   wherein, in Formula (1) above, $R^1$, $R^2$, $R^3$, $R^4$, $R^5$, and $R^6$ each independently represent a hydrogen atom, a linear or branched alkyl or cyclic alkyl group, a linear or branched alkenyl or cyclic alkenyl group, an aryl or heteroaryl group, a hydroxy group, an alkoxy group, an amino group, a monovalent group containing a carbonyl group, an alkylthio group, or an arylthio group; and
   $R^7$ represents a hydrogen atom, a linear or branched alkenyl or cyclic alkenyl group, an aryl or heteroaryl group, a hydroxy group, an alkoxy group, an amino group, a monovalent group containing a carbonyl group, an alkylthio group, or an arylthio group.

2. The methyl methacrylate-containing composition according to claim 1, wherein when the concentration of the component A is MA ($\mu$mol/L), MA is from 1 to 10,000 $\mu$mol/L.

3. The methyl methacrylate-containing composition according to claim 2, wherein the MA is from 10 to 5,000 $\mu$mol/L.

4. The methyl methacrylate-containing composition according to any one of claims 1 to 3, wherein the molecular weight of the component A is 2,000 or less.

5. The methyl methacrylate-containing composition according to any one of claims 1 to 4, further comprising a polymerization inhibitor (component B).

6. The methyl methacrylate-containing composition according to claim 5, wherein when the concentration of the component A is MA ($\mu$mol/L), and the concentration of the component B is MB ($\mu$mol/L), MB/MA is from 0.005 to 30.

7. The methyl methacrylate-containing composition according to claim 6, wherein the MB/MA is from 0.05 to 20.

**8.** The methyl methacrylate-containing composition according to any one of claims 5 to 7, wherein when the concentration of the component B is MB (μmol/L), MB is from 1 to 7,000 μmol/L.

**9.** The methyl methacrylate-containing composition according to any one of claims 6 to 8, wherein the MB is from 10 to 5,000 μmol/L.

**10.** The methyl methacrylate-containing composition according to any one of claims 5 to 9, wherein the component B is at least one polymerization inhibitor selected from the group consisting of a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound.

**11.** The methyl methacrylate-containing composition according to any one of claims 5 to 10, wherein the component B is at least one polymerization inhibitor selected from the group consisting of a phenol compound, an N-oxyl compound, an amine compound, a phosphorus-containing compound, and a sulfur-containing compound.

**12.** The methyl methacrylate-containing composition according to any one of claims 5 to 11, wherein the component B is at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine.

**13.** The methyl methacrylate-containing composition according to any one of claims 1 to 12, wherein the concentration of methyl methacrylate is from 99.8 to 99.99% by mass.

**14.** The methyl methacrylate-containing composition according to any one of claims 1 to 13, wherein the composition does not contain diacetyl, or the concentration of diacetyl contained is 55 μmol/L or less.

**15.** The methyl methacrylate-containing composition according to any one of claims 1 to 14, wherein in the Formula (1), $R^1$, $R^2$, $R^3$, $R^4$, $R^5$, and $R^6$ are each independently a hydrogen atom, a linear or branched $C_{1-5}$ alkyl group or cyclic $C_{1-5}$ alkyl group, a linear or branched $C_{2-5}$ alkenyl group or cyclic $C_{2-5}$ alkenyl group, a $C_{1-12}$ aryl or heteroaryl group, a hydroxy group, a $C_{1-6}$ alkoxy group, an amino group, or a monovalent group containing a carbonyl group, and $R^7$ is a hydrogen atom, a linear or branched. $C_{2-5}$ alkenyl group or cyclic $C_{2-5}$ alkenyl group, a $C_{1-12}$ aryl or heteroaryl group, a hydroxy group, a $C_{1-6}$ alkoxy group, an amino group, a monovalent group containing a carbonyl group.

**16.** The methyl methacrylate-containing composition according to any one of claims 1 to 15, wherein in the Formula (1), $R^1$, $R^2$, $R^3$, $R^4$, $R^5$, and $R^6$ are each independently a hydrogen atom, a linear or branched $C_{1-5}$ alkyl group or cyclic $C_{1-5}$ alkyl group, a hydroxy group, or a $C_{1-6}$ alkoxy group, and $R^7$ is a hydrogen atom, a carboxy group or a $C_{2-6}$ alkoxy carbonyl group.

**17.** The methyl methacrylate-containing composition according to any one of claims 1 to 16, wherein in the Formula (1), $R^1$, $R^2$, $R^3$, $R^4$, $R^5$, and $R^6$ are each independently a hydrogen atom, a methyl group, a t-butyl group, a hydroxy group, or a methoxy group, and $R^7$ is a hydrogen atom, a carboxy group, or a methoxycarbonyl group.

**18.** A method of producing a methyl methacrylate polymer, comprising a step of polymerizing a polymeric composition comprising a methyl methacrylate-containing composition according to any one of claims 1 to 17.

**19.** The method of producing a methyl methacrylate polymer according to claim 18, wherein the polymeric composition comprises a monomer that can be copolymerized with methyl methacrylate.

## Patentansprüche

**1.** Methylmethacrylathaltige Zusammensetzung, umfassend:

Methylmethacrylat und
eine durch die folgende Formel (1) dargestellte alkylsubstituierte Arylverbindung (Komponente A),
wobei die Konzentration von Methylmethacrylat 99 bis 99,99 Masse-% beträgt,

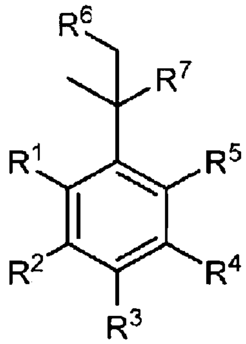

(1)

wobei in der obigen Formel (1) $R^1$, $R^2$, $R^3$, $R^4$, $R^5$ und $R^6$ jeweils unabhängig ein Wasserstoffatom, eine lineare oder verzweigte Alkyl- oder cyclische Alkylgruppe, eine lineare oder verzweigte Alkenyl- oder cyclische Alkenylgruppe, eine Aryl- oder Heteroarylgruppe, eine Hydroxygruppe, eine Alkoxygruppe, eine Aminogruppe, eine einwertige Gruppe, die eine Carbonylgruppe enthält, eine Alkylthiogruppe oder eine Arylthiogruppe darstellen und

$R^7$ ein Wasserstoffatom, eine lineare oder verzweigte Alkenyl- oder cyclische Alkenylgruppe, eine Aryl- oder Heteroarylgruppe, eine Hydroxygruppe, eine Alkoxygruppe, eine Aminogruppe, eine einwertige Gruppe, die eine Carbonylgruppe enthält, eine Alkylthiogruppe oder eine Arylthiogruppe darstellt.

2. Methylmethacrylathaltige Zusammensetzung gemäß Anspruch 1, wobei, wenn die Konzentration der Komponente A MA ($\mu$mol/L) ist, MA 1 bis 10.000 $\mu$mol/L beträgt.

3. Methylmethacrylathaltige Zusammensetzung gemäß Anspruch 2, wobei die MA 10 bis 5.000 $\mu$mol/L beträgt.

4. Methylmethacrylathaltige Zusammensetzung gemäß einem der Ansprüche 1 bis 3, wobei das Molekulargewicht der Komponente A 2.000 oder weniger beträgt.

5. Methylmethacrylathaltige Zusammensetzung gemäß einem der Ansprüche 1 bis 4, ferner umfassend eine Polymerisationsinhibitor (Komponente B).

6. Methylmethacrylathaltige Zusammensetzung gemäß Anspruch 5, wobei, wenn die Konzentration der Komponente A MA ($\mu$mol/L) ist und die Konzentration der Komponente B MB ($\mu$mol/L) ist, MB/MA 0,005 bis 30 beträgt.

7. Methylmethacrylathaltige Zusammensetzung gemäß Anspruch 6, wobei MB/MA 0,05 bis 20 beträgt.

8. Methylmethacrylathaltige Zusammensetzung gemäß einem der Ansprüche 5 bis 7, wobei, wenn die Konzentration der Komponente B MB ($\mu$mol/L) ist, MB 1 bis 7.000 $\mu$mol/L beträgt.

9. Methylmethacrylathaltige Zusammensetzung gemäß einem der Ansprüche 6 bis 8, wobei die MB 10 bis 5.000 $\mu$mol/L beträgt.

10. Methylmethacrylathaltige Zusammensetzung gemäß einem der Ansprüche 5 bis 9, wobei die Komponente B mindestens ein Polymerisationsinhibitor, ausgewählt aus der Gruppe, bestehend aus einer Phenolverbindung, einer Chinonverbindung, einer Nitrobenzolverbindung, einer N-Oxyverbindung, einer Aminverbindung, einer phosphorhaltigen Verbindung, einer schwefelhaltigen Verbindung, einer eisenhaltigen Verbindung, einer kupferhaltigen Verbindung und einer manganhaltigen Verbindung, ist.

11. Methylmethacrylathaltige Zusammensetzung gemäß einem der Ansprüche 5 bis 10, wobei die Komponente B mindestens ein Polymerisationsinhibitor, ausgewählt aus der Gruppe, bestehend aus einer Phenolverbindung, einer N-Oxylverbindung, einer Aminverbindung, einer phosphorhaltigen Verbindung und einer schwefelhaltigen Verbindung, ist.

12. Methylmethacrylathaltige Zusammensetzung gemäß einem der Ansprüche 5 bis 11, wobei die Komponente B mindestens ein Polymerisationsinhibitor, ausgewählt aus der Gruppe, bestehend aus Hydrochinon, 4-Methoxyphenol, 2,4-Dimethyl-6-t-butylphenol, 2,6-Di-t-butyl-4-methylphenol, 4-Hydroxy-2,2,6,6-tetramethylpiperidin-N-oxyl, N,N-Diphenylamin, N-Nitrosodiphenylamin, Triphenylphosphit und Phenothiazin, ist.

**13.** Methylmethacrylathaltige Zusammensetzung gemäß einem der Ansprüche 1 bis 12, wobei die Konzentration von Methylmethacrylat 99,8 bis 99,99 Masse-% beträgt.

**14.** Methylmethacrylathaltige Zusammensetzung gemäß einem der Ansprüche 1 bis 13, wobei die Zusammensetzung kein Diacetyl enthält oder die Konzentration an enthaltenem Diacetyl 55 μmol/L oder weniger beträgt.

**15.** Methylmethacrylathaltige Zusammensetzung gemäß einem der Ansprüche 1 bis 14, wobei in der Formel (1) $R^1$, $R^2$, $R^3$, $R^4$, $R^5$ und $R^6$ jeweils unabhängig ein Wasserstoffatom, eine lineare oder verzweigte $C_{1-5}$-Alkylgruppe oder cyclische $C_{1-5}$-Alkylgruppe, eine lineare oder verzweigte $C_{2-5}$-Alkenylgruppe oder cyclische $C_{2-5}$-Alkenylgruppe, eine $C_{1-12}$-Aryl- oder eine Heteroarylgruppe, eine Hydroxygruppe, eine $C_{1-6}$-Alkoxygruppe, eine Aminogruppe oder eine einwertige Gruppe, die eine Carbonylgruppe enthält, sind und $R^7$ ein Wasserstoffatom, eine lineare oder verzweigte $C_{2-5}$-Alkenylgruppe oder cyclische $C_{2-5}$-Alkenylgruppe, eine $C_{1-12}$-Aryl- oder Heteroarylgruppe, eine Hydroxygruppe, eine $C_{1-6}$-Alkoxygruppe, eine Aminogruppe oder eine einwertige Gruppe, die eine Carboxylgruppe enthält, ist.

**16.** Methylmethacrylathaltige Zusammensetzung gemäß einem der Ansprüche 1 bis 15, wobei in der Formel (1) $R^1$, $R^2$, $R^3$, $R^4$, $R^5$ und $R^6$ jeweils unabhängig ein Wasserstoffatom, eine lineare oder verzweigte $C_{1-5}$-Alkylgruppe oder cyclische $C_{1-5}$-Alkylgruppe, eine Hydroxygruppe oder eine $C_{1-6}$-Alkoxygruppe sind und $R^7$ ein Wasserstoffatom, eine Carboxygruppe oder eine $C_{2-6}$-Alkoxycarbonylgruppe ist.

**17.** Methylmethacrylathaltige Zusammensetzung gemäß einem der Ansprüche 1 bis 16, wobei in der Formel (1) $R^1$, $R^2$, $R^3$, $R^4$, $R^5$ und $R^6$ jeweils unabhängig ein Wasserstoffatom, Methylgruppe, eine t-Butylgruppe, eine Hydroxygruppe oder eine Methoxygruppe sind und $R^7$ ein Wasserstoffatom, eine Carboxygruppe oder eine Methoxycarbonylgruppe ist.

**18.** Verfahren zur Herstellung eines Methylmethacrylatpolymers, umfassend einen Schritt zum Polymerisieren einer polymeren Zusammensetzung, umfassend eine methylmethacrylathaltige Zusammensetzung gemäß einem der Ansprüche 1 bis 17.

**19.** Verfahren zur Herstellung eines Methylmethacrylatpolymers gemäß Anspruch 18, wobei die polymere Zusammensetzung ein Monomer umfasst, das mit Methylmethacrylat copolymerisiert werden kann.

**Revendications**

**1.** Composition à teneur en méthacrylate de méthyle, comprenant :

du méthacrylate de méthyle ; et
un composé aryle substitué par un groupe alkyle représenté par la Formule (1) suivante (composant A),
dans laquelle la concentration en méthacrylate de méthyle est comprise entre 99 % et 99,99 % en masse,

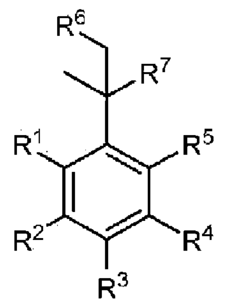

(1)

dans laquelle, dans la Formule (1) ci-dessus, $R^1$, $R^2$, $R^3$, $R^4$, $R^5$ et $R^6$ représentent chacun indépendamment un atome d'hydrogène, un groupe alkyle linéaire ou ramifié ou un groupe cycloalkyle, un groupe alcényle linéaire ou ramifié ou un groupe cycloalcényle, un groupe aryle ou un groupe hétéroaryle, un groupe hydroxy, un groupe alcoxy, un groupe amino, un groupe monovalent contenant un groupe carbonyle, un groupe alkylthio ou un groupe arylthio ; et
$R^7$ représente un atome d'hydrogène, un groupe alcényle linéaire ou ramifié ou un groupe cycloalcényle, un groupe aryle ou un groupe hétéroaryle, un groupe hydroxy, un groupe alcoxy, un groupe amino, un groupe

monovalent contenant un groupe carbonyle, un groupe alkylthio ou un groupe arylthio.

**2.** Composition à teneur en méthacrylate de méthyle selon la revendication 1, dans laquelle, lorsque la concentration en composant A est MA (μmol/L), MA est comprise entre 1 μmol/L et 10 000 μmol/L.

**3.** Composition à teneur en méthacrylate de méthyle selon la revendication 2, dans laquelle la MA est comprise entre 10 μmol/L et 5 000 μmol/L.

**4.** Composition à teneur en méthacrylate de méthyle selon l'une quelconque des revendications 1 à 3, dans laquelle le poids moléculaire du composant A est inférieur ou égal à 2 000.

**5.** Composition à teneur en méthacrylate de méthyle selon l'une quelconque des revendications 1 à 4, comprenant en outre un inhibiteur de polymérisation (composant B).

**6.** Composition à teneur en méthacrylate de méthyle selon la revendication 5, dans laquelle, lorsque la concentration en composant A est MA (μmol/L), et la concentration en composant B est MB (μmol/L), MB/MA est compris entre 0,005 et 30.

**7.** Composition à teneur en méthacrylate de méthyle selon la revendication 6, dans laquelle le MB/MA est compris entre 0,05 et 20.

**8.** Composition à teneur en méthacrylate de méthyle selon l'une quelconque des revendications 5 à 7, dans laquelle, lorsque la concentration en composant B est MB (μmol/L), MB est comprise entre 1 μmol/L et 7 000 μmol/L.

**9.** Composition à teneur en méthacrylate de méthyle selon l'une quelconque des revendications 6 à 8, dans laquelle la MB est comprise entre 10 μmol/L et 5 000 μmol/L.

**10.** Composition à teneur en méthacrylate de méthyle selon l'une quelconque des revendications 5 à 9, dans laquelle le composant B est au moins un inhibiteur de polymérisation choisi dans le groupe constitué d'un composé phénol, d'un composé quinone, d'un composé nitrobenzène, d'un composé N-oxyle, d'un composé amine, d'un composé contenant du phosphore, d'un composé contenant du soufre, d'un composé contenant du fer, d'un composé contenant du cuivre et d'un composé contenant du manganèse.

**11.** Composition à teneur en méthacrylate de méthyle selon l'une quelconque des revendications 5 à 10, dans laquelle le composant B est au moins un inhibiteur de polymérisation choisi dans le groupe constitué d'un composé phénol, d'un composé N-oxyle, d'un composé amine, d'un composé contenant du phosphore et d'un composé contenant du soufre.

**12.** Composition à teneur en méthacrylate de méthyle selon l'une quelconque des revendications 5 à 11, dans laquelle le composant B est au moins un inhibiteur de polymérisation choisi dans le groupe constitué d'hydroquinone, de 4-méthoxyphénol, de 2,4-diméthyl-6-t-butylphénol, de 2,6-di-t-butyl-4-méthylphénol, de 4-hydroxy-2,2,6,6-tétraméthylpipéridine-N-oxyl, de N,N-diphénylamine, de N-nitrosodiphénylamine, de phosphite de triphényle et de phénothiazine.

**13.** Composition à teneur en méthacrylate de méthyle selon l'une quelconque des revendications 1 à 12, dans laquelle la concentration en méthacrylate de méthyle est comprise entre 99,8 % et 99,99 % en masse.

**14.** Composition à teneur en méthacrylate de méthyle selon l'une quelconque des revendications 1 à 13, dans laquelle la composition ne contient pas de diacétyle, ou la concentration en diacétyle contenue est de 55 μmol/L ou moins.

**15.** Composition à teneur en méthacrylate de méthyle selon l'une quelconque des revendications 1 à 14, dans laquelle, dans la Formule (1), $R^1$, $R^2$, $R^3$, $R^4$, $R^5$ et $R^6$ sont chacun indépendamment un atome d'hydrogène, un groupe alkyle linéaire ou ramifié en $C_{1-5}$ ou un groupe cycloalkyle en $C_{1-5}$, un groupe alcényle linéaire ou ramifié en $C_{2-5}$ ou un groupe cycloalcényle en $C_{2-5}$, un groupe aryle en $C_{1-12}$ ou un groupe hétéroaryle, un groupe hydroxy, un groupe alcoxy en $C_{1-6}$, un groupe amino, ou un groupe monovalent contenant un groupe carbonyle, et $R^7$ est un atome d'hydrogène, un groupe alcényle linéaire ou ramifié en $C_{2-5}$ ou un groupe cycloalcényle en $C_{2-5}$, un groupe aryle en $C_{1-12}$ ou un groupe hétéroaryle, un groupe hydroxy, un groupe alcoxy en $C_{1-6}$, un groupe amino, un groupe monovalent contenant un groupe carbonyle.

**16.** Composition à teneur en méthacrylate de méthyle selon l'une quelconque des revendications 1 à 15, dans laquelle, dans la Formule (1), $R^1$, $R^2$, $R^3$, $R^4$, $R^5$ et $R^6$ sont chacun indépendamment un atome d'hydrogène, un groupe alkyle linéaire ou ramifié en $C_{1-5}$ ou un groupe cycloalkyle en $C_{1-5}$, un groupe hydroxy, ou un groupe alcoxy en $C_{1-6}$, et $R^7$ est un atome d'hydrogène, un groupe carboxy ou un groupe alcoxycarbonyle en $C_{2-6}$.

**17.** Composition à teneur en méthacrylate de méthyle selon l'une quelconque des revendications 1 à 16, dans laquelle dans la Formule (1), $R^1$, $R^2$, $R^3$, $R^4$, $R^5$ et $R^6$ sont chacun indépendamment un atome d'hydrogène, un groupe méthyl, un groupe t-butyle, un groupe hydroxy ou un groupe méthoxy, et $R^7$ est un atome d'hydrogène, un groupe carboxy ou un groupe méthoxycarbonyle.

**18.** Procédé de fabrication d'un polymère de méthacrylate de méthyle, comprenant une étape de polymérisation d'une composition polymère comprenant une composition à teneur en méthacrylate de méthyle selon l'une quelconque des revendications 1 à 17.

**19.** Procédé de fabrication d'un polymère de méthacrylate de méthyle selon la revendication 18, dans lequel la composition polymère comprend un monomère qui peut être copolymérisé avec le méthacrylate de méthyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004155757 A
- JP 2005502695 A
- JP H10504553 A
- JP 2002533309 A
- JP 2002513034 A
- CN 1331669
- JP 2019127436 A
- JP 2007045803 A


### Non-patent literature cited in the description


- Development of Catalyst for Producing Methyl Methacrylate. **TORU KURODA**. Catalysts & Catalysis. Catalysis Society of Japan, 2003, vol. 45, 366-371
- On the Functions of Polymerization Inhibitors. **TAKAYUKI OTSU**. Synthetic Organic Chemistry. The Society of Synthetic Organic Chemistry, 1975, vol. 33, 634-640